(19) Europäisches Patentamt
European Patent Office
Office européen des brevets

(11) Publication number : 0 394 927 B1

(12) EUROPEAN PATENT SPECIFICATION

(45) Date of publication of patent specification : 08.12.93 Bulletin 93/49

(51) Int. Cl.$^5$ : C07C 69/653, C07C 67/08

(21) Application number : 90107669.5

(22) Date of filing : 23.04.90

# (54) Process for preparing methacrylates of fluorinated alcohols.

(30) Priority : 24.04.89 IT 2026989

(43) Date of publication of application : 31.10.90 Bulletin 90/44

(45) Publication of the grant of the patent : 08.12.93 Bulletin 93/49

(84) Designated Contracting States : BE DE FR GB NL

(56) References cited :
DE-A- 1 495 075
INDUSTRIAL AND ENGINEERING CHEMISTRY, vol. 42, no. 5, 29 April 1950, pages 768-776; J.M. CHURCH et al.: "Methyl methacrylate from methallyl alcohol"

(73) Proprietor : AUSIMONT S.p.A.
31 Foro Buonaparte
I-20121 Milano (IT)

(72) Inventor : Gregorio, Guglielmo, Dr.
7/2 Via Fabriano
I-20161 Milan (IT)
Inventor : Roberti, Lamberto
38, Via Borgazzi
I-20052 Monza, Milan (IT)
Inventor : Strepparola, Ezio, Dr.
6, V.le Partigiano
I-24097 Treviglio, Bergamo (IT)

(74) Representative : Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-80803 München (DE)



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The object of the present invention is a method for preparing methacrylates of fluorinated alcohols by direct esterification of said alcohols with methacrylic acid.

The methacrylates of fluorinated alcohols which exhibit a high degree of purity are particularly suitable for the production of polymers or copolymers with methacrylates of non-fluorinated alcohols, for example methyl methacrylate. These highly transparent polymeric products have a lower refractive index than the polymethyl methacrylate itself, as it is required for particular applications such as, for example, the coating of optical fibres.

In consideration of the high costs of the fluorinated alcohols it is essential to have available a method for the preparation of methacrylates which does not require an excess of alcohol and which, besides providing high yields, permits in the first place to obtain an easily purifiable ester.

Several esterification methods are described in the literature, which, however, if applied to the synthesis of methacrylates of fluorinated alcohols, are not too suitable for the above purpose, both because the yields are very low and because the recovery of the products and the unreacted alcohols involves difficult operations.

J. Hollander and C. Woolf have synthesized hexafluoroisopropyl methacrylate by reacting hexafluoroisopropanol with methacryloyl chloride in the presence of pyridine, as described in USA-3,177,185. The yields are very low, about 20% with respect to the alcohol utilized.

M. Kojima et al. (Iyo Kizai Kenkyusho Hokoku, 1983, 17, 37-43; see C.A. 101, 116698 s) have synthesized trifluoroethyl methacrylate and hexafluoroisopropyl methacrylate by reacting methacryloyl chloride with trifluoroethyl alcohol and hexafluoroisopropyl alcohol, respectively, in the presence of triethylamine. In this case, too, the yields are not too satisfactory, i.e., 14-15%.

The direct esterification, i.e. the reaction between acid and alcohol, according to the art, seemed not suitable for alcohols of acidic nature, as are the fluorinated alcohols. In fact, the equilibrium of the type:

$$R_fCH_2OH + CH_2 = C(CH_3)COOH \rightleftarrows R_fCH_2\text{-}O\text{-}CO\text{-}C(CH_3)=CH_2 + H_2O$$

where $R_f$ represents a fluorinated radical, is not shifted towards the formation of the ester.

In the field of esterification the use of a strong dehydrating agent, such as sulphuric acid or phosphoric anhydride generally is not free from drawbacks, as these dehydrating agents react with most of the alcohols, thereby giving rise to ethers or olefins. Also many organic acids react with formation of ketenes or anhydrides and the unsaturated acids tend to polymerize.

Consequently, the use of a dehydrating agent cannot be considered to be suitable for the preparation of any ester.

In particular, phosphoric anhydride has been used very seldom for esterification reactions. As it is stated by A. Banerjee et al., J. Org. Chem. 1983, 48, 3106-08, the only example of the use of phosphoric anhydride in such a reaction, before said authors, is reported in the article by J.M. Church and L. Lynn, Ind. Eng. Chem. 1950, 42, 768, which describes the esterification of methacrylic acid with methanol in the presence of phosphoric anhydride, operating with an alcohol/acid molar ratio equal to 2.5.

A. Banerjee et al. describe, in the above article, the preparation of esters starting from various acids and alcohols in the presence of phosphoric anhydride, operating with a considerable excess of alcohol. The authors state that with certain acids the reaction proceeds with high yields, while with other acids the reaction takes place with very low yields or does not take place at all, even if a large excess of phosphoric anhydride is employed, which is indicative of the unforeseeable nature of the reaction. Furthermore, according to said authors, the method is not suitable for the esterification of secondary or tertiary alcohols.

Anyhow, no examples of direct esterification of acids with fluorinated alcohols can be found in the literature.

The object of the present invention is to provide a process which permits to obtain methacrylates of fluorinated alcohols with a high degree of purity, by direct esterification of said alcohols with methacrylic acid, with high yields with respect to the alcohols.

This object is achieved by the process of the present invention, which comprises the reaction of a fluorinated alcohol with an excess of methacrylic acid, in the presence of (preferably an excess of) phosphoric anhydride, at a temperature ranging from about 30° to about 150°C, preferably from about 80° to about 105°C.

The molar ratio of methacrylic acid to hydroxyl groups of the alcohol preferably ranges from 1 to 10, particularly from 2 to 5.

The phosphoric anhydride employed should be present in activated form, i.e. in a finely crystalline form. The molar ratio phosphoric anhydride/hydroxyl groups preferably ranges from 0.2 to 5.

The esterification method of the present invention may be used for primary, secondary and tertiary alcohols having the general formula:

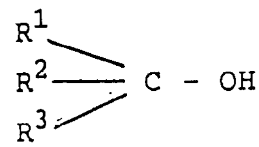

wherein $R^1$, $R^2$ and $R^3$, the same or different from each other, represent a perfluoroalkyl radical, an aromatic non-fluorinated hydrocarbon radical or hydrogen, at least one of $R^1$, $R^2$ and $R^3$ being a perfluoroalkyl radical.

Preferably $R^1$, $R^2$ and $R^3$ are $C_1$-$C_4$ perfluoroalkyl, phenyl, tolyl or xylyl radicals.

As examples of primary alcohols $CF_3CH_2OH$ and higher hornologes thereof may be mentioned. An example of a secondary fluorinated alcohol is $CF_3$-CHOH-$CF_3$ and an example of a tertiary fluorinated alcohol is an alcohol of formula

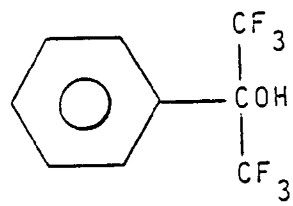

The process of the present invention may also be applied for primary, secondary and tertiary fluorinated diols and polyols, such as:

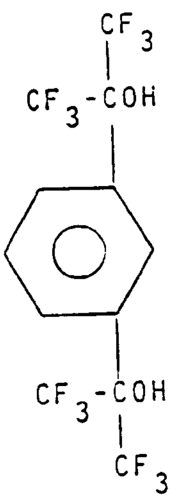

The esterification method of the present invention may furthermore be applied to alcohols having a perfluoropolyether chain of the type:

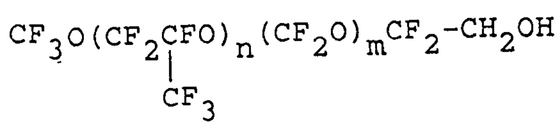

The latter alcohols are generally utilized as mixtures in which n and m are differently distributed integers so as to result in average molecular weights of from about 400 to about 2,500, and they may be obtained according to the methods described in US-A-3,513,203; 3,847,978; and 3,810,174. The present method may also be applied to diols with a perfluoropolyether chain having two end groups $CH_2OH$, e.g., of the type $HOCH_2$-$CF_2O(CF_2CF_2O)_n(CF_2O)_mCF_2CH_2OH$ such as those obtainable by the method described in US-A-3,810,874, and with n and m being integers such as to result in mixtures having an average molecular weight from about 500 to about 5,000.

The isolation of the product from the reaction mixture usually is relatively simple. In the case of methacrylates of alcohols such as trifluoroethanol or hexafluoroisopropanol and, in general, of products having a

significantly lower boiling point than that of methacrylic acid, after separation of phosphoric acid and of residual phosphoric anhydride by decantation, distillation may be carried out directly.

In the case of high-boiling fluorinated alcohols, methacrylic acid, phosphoric acid and residual phosphoric anhydride may be separated by washing with water or with water and sodium bicarbonate or, particularly for the high molecular weight alcohols which are insoluble in common organic solvents, methacrylic acid and the other undesired products may be removed by washing with acetone.

If particular measures are taken, a few of the high-boiling esters can be distilled under vacuum.

The phosphoric anhydride utilized in these preparations should be in the activated form, i.e. in the form of a finely crystalline powder.

Furthermore, it is preferable to work in the presence of polymerization inhibitors, such as di-tert.-butyl-p-cresol, tert.-butyl-catechol, p-methoxyphenol,etc.

As already mentioned, the obtained products easily reach the degree of purity required for polymerizations. For example, the mixture of methyl methacrylate and hexafluoroisopropyl methacrylate containing from about 10% to about 40% of hexafluoroisopropyl methacrylate, prepared in the manner described above and directly distilled from the crude product, can be polymerized, resulting in a high molecular weight copolymer which does not give rise to phase segregation and therefore appears homogeneous and transparent.

The same is true for the trifluoroethanol methacrylate prepared by the present method.

The method of the present invention is specific for the esterification of methacrylic acid with fluorinated alcohols and cannot be applied to the esterification of other unsaturated acids. In fact, in the case of other unsaturated acids, for example acrylic acid, the ester yields are low and oligomers of the acid are formed.

The following examples are given for merely illustrative purposes.

## EXAMPLE 1

416 g of methacrylic acid, 3 g of di-tert.-butyl-p-cresol (polymerization inhibitor) and 101 g of phosphoric anhydride were poured into a one-liter flask equipped with mechanical stirrer, thermometer, heating and cooling system. The whole mixture was stirred and 270 g of hexafluoroisopropanol were added. Thereafter the resulting mixture was heated to 100°C for 75 minutes, cooled and the phases were separated. From the methacrylic phase, by vacuum distillation, 328.4 g of pure hexafluoro-isopropanol methacrylate (b.p. 47°C at 100 mm Hg) were recovered, corresponding to a yield of 86.4%, based on the fluorinated alcohol.

## EXAMPLE 2

155 g of methacrylic acid, 1.5 g of tert.-butyl-p-cresol and 38 g of phosphoric anhydride were poured into a 250 ml flask equipped with mechanical stirrer, thermometer, heating and cooling system. The mixture was stirred and 60 g of trifluoroethanol were added. Then the resulting mixture was heated to 60°C for 60 minutes, cooled and the phases were separated. From the methacrylic phase, by distillation under vacuum, 84.8 g of pure trifluoroethanol methacrylate (b.p. 40°C at 50 mm Hg) were recovered. The yield was 84%.

## EXAMPLE 3 (comparative test)

44 g of arylic acid, 1.5 g of di-tert.-butyl-p-cresol and 13.8 g of phosphoric anhydride were poured into a 250 ml flask equipped with mechanical stirrer, thermometer, cooling and heating system. The mixture was stirred and 33.5 g of hexafluoroisopropanol were added. The resulting mixture was heated to 100°C. After 60 minutes, all of the acrylic acid had been consumed, while 30% of hexafluoroisopropyl alcohol had remained unconverted. Furthermore, several high-boiling products were present. The GCC/SM analysis revealed that they were acrylic acid oligomers, a part of which was esterified with hexafluoroisopropanol.

From the foregoing it is apparent that the method of the present invention is specific for methacrylic acid and cannot be used for other unsaturated acids.

## EXAMPLE 4

A mixture of alcohols with a perfluoropolyether chain of general formula:

$$CF_3O(CF_2-\underset{\underset{CF_3}{|}}{C}FO)_n(CF_2O)_mCF_2CH_2OH,$$

and an average molecular weight of 600, was subjected to esterification with methacrylic acid. 70 g of this mixture (0.117 moles of alcohol) were treated with 50 g (0.58 moles) of methacrylic acid, 8.5 g (0.06 moles) of phosphoric anhydride and 0.5 g of tert.-butyl catechol as polymerization inhibitor.

The mixture was heated to 100°C under stirring for 1 hour. After cooling, it was washed with water, thereby separating the phases; then it was repeatedly washed with an aqueous solution of sodium bicarbonate.

The remaining organic product consisted of 69.5 g of methacrylic esters of the introduced alcohols. The liquid, slightly colored and rendered limpid by filtration, exhibited an I.R. (infrared) spectrum in which the bands due to alcohol and to methacrylic acid were missing. An intense band at 1745 $cm^{-1}$ due to the ester was present.

EXAMPLE 5

A mixture of difunctional alcohols with perfluoropolyether chain terminated with two $-CH_2OH$ groups of general formula:

$$HOCH_2CF_2O(CF_2CF_2O)_m(CF_2O)_nCF_2CH_2OH$$

having an average molecular weight of 4,000, prepared according to US-A-3,810,874, was utilized for the esterification with methacrylic acid.

200 g of the diol mixture were added to 65 g (0.75 moles) of methacrylic acid, 1.5 g of p-methoxyphenol as polymerization inhibitor and 8 g of phosphoric anhydride.

The resulting mixture was heated to 80°C for 20 hours under intense stirring, checking from time to time the conversion degree on a sample by means of I.R. spectrometry and $^{19}F$-N.M.R.

After 20 hours, the N.M.R. signals of the starting alcohol were strongly reduced, while the I.R. band at 1745 $cm^{-1}$ due to the esters was intense. In order to obtain an absolutely pure product it is advisable to add further 8 g of phosphoric anhydride to the mixture and to continue heating for further 10 hours.

After cooling and decantation, the product was repeatedly washed with acetone (in which it was insoluble) in order to remove the methacrylic acid excess, whereafter it was dried under vacuum. N.M.R. signals due to the $CF_2CH_2OH$ groups were no longer detectable and the ester number corresponded to the equivalent weight of the diol.

The product can be further purified by washing with ethyl ether.

## Claims

1. Process for preparing methacrylates of fluorinated alcohols, wherein the fluorinated alcohol is reacted with an excess of methacrylic acid in the presence of phosphoric anhydride, at a temperature of from about 30 to about 150°C.

2. Process according to claim 1, wherein the fluorinated alcohol is a primary, secondary or tertiary alcohol of formula:

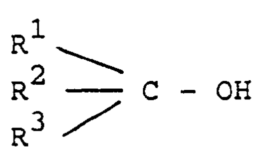

wherein $R^1$, $R^2$ and $R^3$ independently represent a perfluoroalkyl radical, a non-fluorinated aromatic hydrocarbon radical or hydrogen, at least one of $R^1$, $R^2$ and $R^3$ being a perfluoroalkyl radical.

3. Process according to claim 2, wherein $R^1$, $R^2$ and $R^3$, the same or different from each other, are $C_1$-$C_4$-perfluoroalkyl, phenyl, tolyl and/or xylyl radicals.

4. Process according to claim 2, wherein the fluorinated alcohol is trifluoroethanol or hexafluoroisopropanol.

5. Process according to claim 2, wherein the fluorinated alcohol is a mixture of alcohols with perfluoropolyether chain of formula:

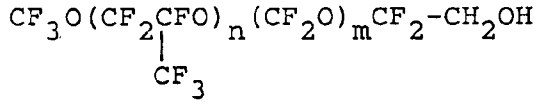

in which n and m are integers such that the average molecular weight ranges from about 400 to about 2,500.

6. Process according to claim 1, wherein the fluorinated alcohol is a diol or a polyol.

7. Process according to claim 6, wherein the fluorinated alcohol is a mixture of diols of formula:

$$HOCH_2\text{-}CF_2O(CF_2\text{-}CF_2O)_n(CF_2O)_mCF_2\text{-}CH_2OH$$

in which n and m are integers such that the average molecular weight ranges from about 500 to about 5,000.

8. Process according to one or more of the preceding claims, wherein the molar ratio of methacrylic acid to OH groups of the alcohol ranges from 1 to 10.

9. Process according to one or more of the preceding claims, wherein the molar ratio of phosphoric anhydride to OH groups of the alcohol ranges from 0.2 to 5.

10. Process according to one or more of the preceding claims, wherein the temperature ranges from about 80 to about 105°C.

**Patentansprüche**

1. Verfahren zur Herstellung von Methacrylaten von fluorierten Alkoholen, in welchem der fluorierte Alkohol mit einem Überschuß an Methacrylsäure in Anwesenheit von Phosphorsäureanhydrid bei einer Temperatur von etwa 30 bis etwa 150°C umgesetzt wird.

2. Verfahren nach Anspruch 1, in welchem der fluorierte Alkohol ein primärer, sekundärer oder tertiärer Alkohol der Formel:

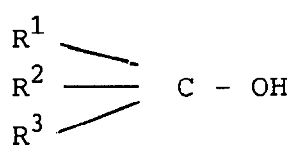

ist, worin $R^1$, $R^2$ und $R^3$ unabhängig einen Perfluoralkylrest, einen nicht-fluorierten aromatischen Kohlenwasserstoffrest oder Wasserstoff darstellen, wobei wenigstens eines von $R^1$, $R^2$ und $R^3$ ein Perfluoralkylrest ist.

3. Verfahren nach Anspruch 2, worin $R^1$, $R^2$ und $R^3$, gleich oder verschieden voneinander, $C_1$-$C_4$-Perfluoralkyl-, Phenyl-, Tolyl- und/oder Xylylreste sind.

4. Verfahren nach Anspruch 2, in welchem der fluorierte Alkohol Trifluorethanol oder Hexafluorisopropanol ist.

5. Verfahren nach Anspruch 2, in welchem der fluorierte Alkohol eine Mischung von Alkoholen mit Perfluorpolyetherkette der Formel:

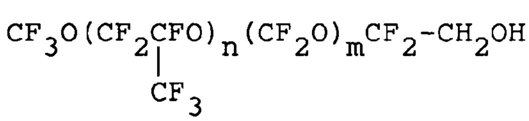

ist, in welcher n und m solche ganze Zahlen sind, daß das durchschnittliche Molekulargewicht im Bereich

von etwa 400 bis etwa 2500 liegt.

6. Verfahren nach Anspruch 1, in welchem der fluorierte Alkohol ein Diol oder ein Polyol ist.

7. Verfahren nach Anspruch 6, in welchem der fluorierte Alkohol eine Mischung von Diolen der Formel:

$$HOCH_2\text{-}CF_2O(CF_2\text{-}CF_2O)_n(CF_2O)_mCF_2\text{-}CH_2OH$$

ist, in welcher n und m solche ganze Zahlen sind, daß das durchschnittliche Molekulargewicht im Bereich von etwa 500 bis etwa 5000 liegt.

8. Verfahren nach einem oder mehreren der vorangehenden Ansprüche, in welchem das Molverhältnis von Methacrylsäure zu OH-Gruppen des Alkohols im Bereich von 1 bis 10 liegt.

9. Verfahren nach einem oder mehreren der vorangehenden Ansprüche, in welchem das Molverhältnis von Phosphorsäureanhydrid zu OH-Gruppen des Alkohols im Bereich von 0,2 bis 5 liegt.

10. Verfahren nach einem oder mehreren der vorangehenden Ansprüche, in welchem die Temperatur im Bereich von etwa 80 bis etwa 105°C liegt.

## Revendications

1. Procédé pour préparer des méthacrylates d'alcools fluorés, dans lequel l'alcool fluoré est traité avec un excès d'acide méthacrylique en présence d'anhydride phosphorique, à une température comprise entre environ 30° et environ 150°C.

2. Procédé selon la revendication 1, dans lequel l'alcool fluoré est un alcool primaire, secondaire ou tertiaire représenté par la formule générale:

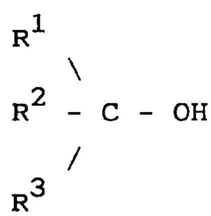

dans laquelle:
$R^1$, $R^2$ et $R^3$ représentent indépendamment les uns des autres un radical perfluoroalkyle, un radical hydrocarboné aromatique non fluoré ou un atome d'hydrogène, l'un au moins des radicaux $R^1$, $R^2$ et $R^3$ étant un radical perfluoroalkyle.

3. Procédé selon la revendication 2, dans lequel $R^1$, $R^2$ et $R^3$ identiques ou différents les uns des autres, sont des radicaux perfluoroalkyles en $C_{1-4}$, phényle, tolyle et/ou xylyle.

4. Procédé selon la revendication 2, dans lequel l'alcool fluoré est le trifluoroéthanol ou l'hexafluoroisopropanol.

5. Procédé selon la revendication 2, dans lequel l'alcool fluoré est un mélange d'alcools ayant une chaîne perfluoropolyéther du type:

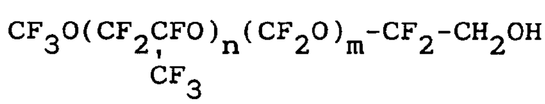

dans lesquels:
n et m sont des entiers tels que le poids moléculaire moyen soit compris entre environ 400 et environ 2.500.

6. Procédé selon la revendication 1, dans lequel l'alcool est un diol ou un polyol.

7. Procédé selon la revendication 1, dans lequel l'alcool fluoré est un mélange d'alcools ayant une chaîne perfluoropolyéther du type:

$$HOCH_2\text{-}CF_2O(CF_2CF_2O)_n(CF_2O)_mCF_2CH_2OH$$

dans lesquels:
n et m sont des entiers tels que le poids moléculaire moyen soit compris entre environ 500 et environ 5.000.

8. Procédé selon l'une ou plusieurs des revendications précédentes, dans lequel le rapport molaire acide méthacrylique/groupes OH de l'alcool est compris entre 1 et 10.

9. Procédé selon l'une ou plusieurs des revendications précédentes, dans lequel le rapport molaire anhydride phosphorique/groupes OH de l'alcool est compris entre 0,2 et 5.

10. Procédé selon l'une ou plusieurs des revendications précédentes, dans lequel la température est comprise entre environ 80 et environ 105°C.